# EUROPEAN PATENT SPECIFICATION

(11) **EP 2 780 081 B1**
(45) Date of publication and mention of the grant of the patent: **10.05.2017**
(21) Application number: 12779241.4
(22) Date of filing: 09.10.2012
(51) Int. Cl.: A61B 18/00, A61B 18/18, A61B 18/20, A61N 7/00, A61N 7/02

(54) **SHAFT WITH ULTRASOUND TRANSDUCERS FOR NERVE MODULATION**
SCHAFT MIT ULTRASCHALLWANDLERN ZUR NEUROMODULATION
TIGE AVEC DES TRANSDUCTEURS À ULTRASONS POUR LA NEUROMODULATION

(30) Priority: 15.11.2011 US 201161560068 P
(43) Date of publication of application: 24.09.2014
(73) Proprietor: Boston Scientific Scimed, Inc., Maple Grove, MN 55311-1566 (US)
(72) Inventor: SMITH, Scott R., Chaska, MN 55318 (US)
(74) Representative: Vossius & Partner Patentanwälte Rechtsanwälte mbB
(86) International application number: PCT/US2012/059360
(87) International publication number: WO 2013/074218

(56) References cited:
- US-A1- 2009 076 409
- US-A1- 2009 230 822
- US-A1- 2009 254 134
- US-A1- 2010 217 162

## Description

### Technical Field

The present disclosure relates to a system for nerve modulation such as ablation of nerve tissue or other destructive modulation through the walls of blood vessels.

### Background

Certain treatments require the temporary or permanent interruption or modification of select nerve function. One example treatment is renal nerve ablation which is sometimes used to treat conditions related to congestive heart failure or hypertension. The kidneys produce a sympathetic response to congestive heart failure, which, among other effects, increases the undesired retention of water and/or sodium. Ablating some of the nerves running to the kidneys may reduce or eliminate this sympathetic function, which may provide a corresponding reduction in the associated undesired symptoms.

Many nerves (and nervous tissue such as brain tissue), including renal nerves, run along the walls of or in close proximity to blood vessels and thus can be accessed intravascularly through the walls of the blood vessels. In some instances, it may be desirable to ablate perivascular renal nerves using ultrasound energy. In other instances, it may be desirable to ablate perivascular renal nerves using a radio frequency (RF) electrode. However, systems utilizing a single ultrasound transducer or RF electrode may require repositioning to complete the desired ablation. The document US 2009/076409 A1 discloses a system for nerve modulation including two ultrasound transducers provided at the end of a shaft, each transducer positioned at a different angle with respect to the shaft axis. It may be desirable to provide for alternative systems for intravascular nerve modulation.

### Summary

The disclosure is directed to a system for performing nerve ablation.

Accordingly, a system for nerve modulation is disclosed that includes an elongate shaft having a proximal end region and a distal end region. Two or more ultrasound transducers are positioned adjacent to the distal end region. The transducers may be arranged in an array such as a longitudinal array or a radial array. Each of the transducers includes a first side surface and a second side surface. Acoustic energy can be radiated from the first and second surfaces simultaneously.

### Brief Description of the Drawings

The invention may be more completely understood in consideration of the following detailed description in connection with the accompanying drawings, in which:
Figure 1 is a schematic view illustrating a renal nerve modulation system in situ.
Figure 2 is a perspective view of a distal end of an illustrative renal nerve modulation system.
Figure 3 is an end view of the illustrative renal nerve modulation system of Figure 2.
Figure 4 is a cross-section of one of the ultrasound transducers of the illustrative renal nerve modulation system shown in Figure 2.
Figure 5 is a perspective view of a distal end of an illustrative renal nerve modulation system.
Figure 6 is an alternative perspective view of the illustrative renal nerve modulation system of Figure 5.
Figure 7 is perspective view of a distal end of an illustrative renal nerve modulation system.
Figure 8 is an end view of the illustrative renal nerve modulation system of Figure 7.
Figure 9 is an end view of another illustrative renal nerve modulation system.
Figure 10 is an end view of another illustrative renal nerve modulation system. Specifics of the ivention have been shown by way of example in the drawings and will be described in detail. It should be understood, however, that the intention is not to limit aspects of the invention to the particular embodiments described.

### Detailed Description

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary.

While the system described herein is discussed relative to renal nerve modulation, it is contemplated that the system may be used in other applications where nerve modulation and/or ablation are desired, such as, but not limited to: blood vessels, urinary vessels, or in other tissues via trocar and cannula access. In some instances, it may be desirable to ablate perivascular renal nerves with ultrasound ablation.

In some instances, ultrasound ablation may be used to ablate a desired target region. In some instances, a nerve ablation system including a single ultrasound transducer may require repositioning to provide circumferential ablation and/or ablation along a length of a vessel. Ablating a series of locations one after the other may allow the target tissue to cool between burns. This may increase the length of the time required to perform the procedure. An ablation system including multiple transducers may complete ablation of the target tissue without requiring repositioning.

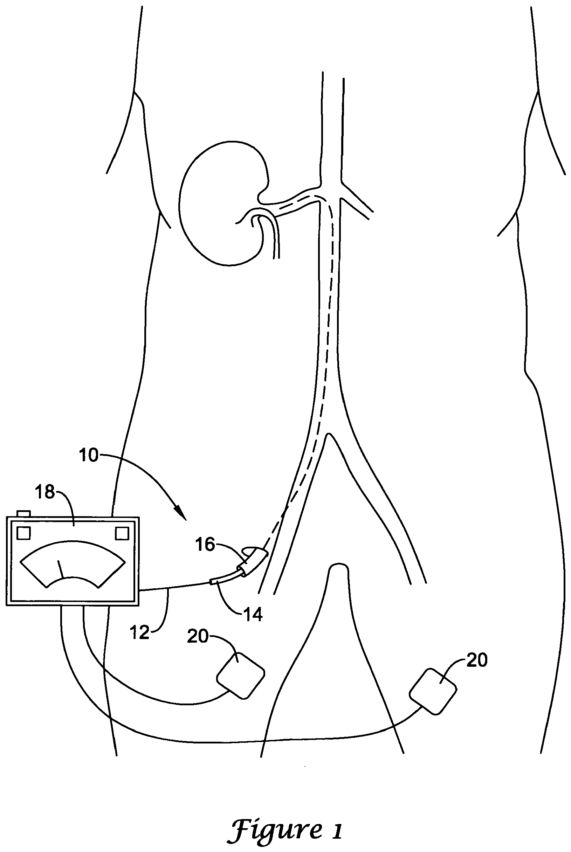

Figure 1 is a schematic view of an illustrative renal nerve modulation system 10 in situ. System 10 may include an element 12 for providing power to a transducer disposed adjacent to, about, and/or within a central elongate shaft 14 and, optionally, within a sheath 16, the details of which can be better seen in subsequent figures. A proximal end of element 12 may be connected to a control and power element 18, which supplies the necessary electrical energy to activate the one or more transducers (when ultrasound ablation is being performed) at or near a distal end of the element 12. The control and power element 18 may include monitoring elements to monitor parameters such as power, temperature, voltage, and/or frequency and other suitable parameters as well as suitable controls for performing the desired procedure. In some instances, the power element 18 may control an ultrasound transducer. An ultrasound transducer may be configured to operate at a frequency of approximately 9 - 10 megahertz (MHz). It is contemplated that any desired frequency may be used, for example, from 1 - 20MHz. However, it is contemplated that frequencies outside this range may also be used, as desired. It is contemplated that different types of energy outside the ultrasound spectrum may be used as desired, for example, but not limited to radiofrequency (RF), microwave, or laser. In some instances, such as when RF ablation is performed, return electrode patches 20 may be supplied on the legs or at another conventional location on the patient's body to complete the circuit, although this is not required.

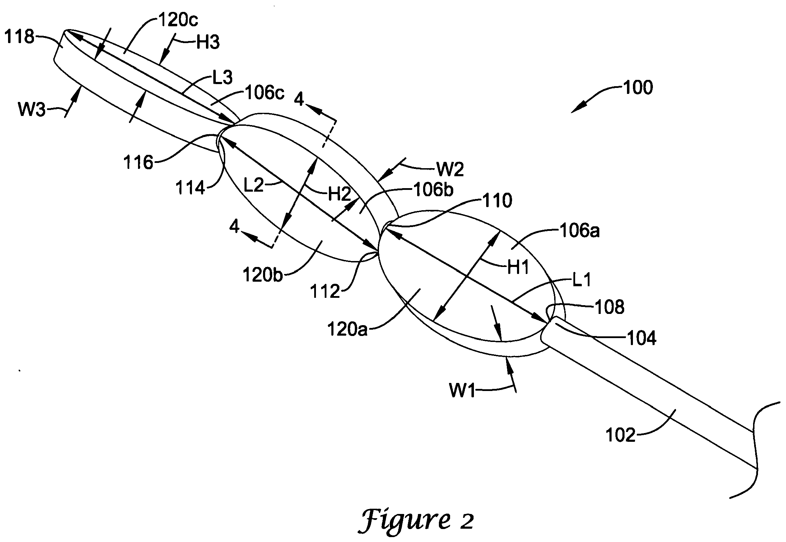

Figure 2 is a perspective view of a distal end of an illustrative renal nerve modulation system 100. The system 100 includes an elongate shaft 102 having a distal end 104. The elongate shaft 102 extends proximally from the distal end 104 to a proximal end (not shown) configured to remain outside of a patient's body. The proximal end of the elongate shaft 102 may include a hub attached thereto for connecting other diagnostic and/or treatment devices or for providing a port for facilitating other interventions.

It is contemplated that the stiffness of the elongate shaft 102 may be modified to form modulation systems 100 for use in various vessel diameters. The elongate shaft 102 may further include one or more lumens extending therethrough. For example, the elongate shaft 102 may include a guidewire lumen and/or one or more auxiliary lumens. The lumens may be configured in any suitable way such as those ways commonly used for medical device. For example, the guidewire lumen may extend the entire length of the elongate shaft 102 such as in an over-the-wire catheter or may extend only along a distal portion of the elongate shaft 102 such as in a single operator exchange (SOE) catheter. These cases are examples of some possible configurations. While not explicitly shown, the modulation system 100 may further include temperature sensors/wire, an infusion lumen, radiopaque marker bands, fixed guidewire tip, a guidewire lumen, external sheath and/or other components may be incorporated to facilitate the use and advancement of the system 100 within the vasculature.

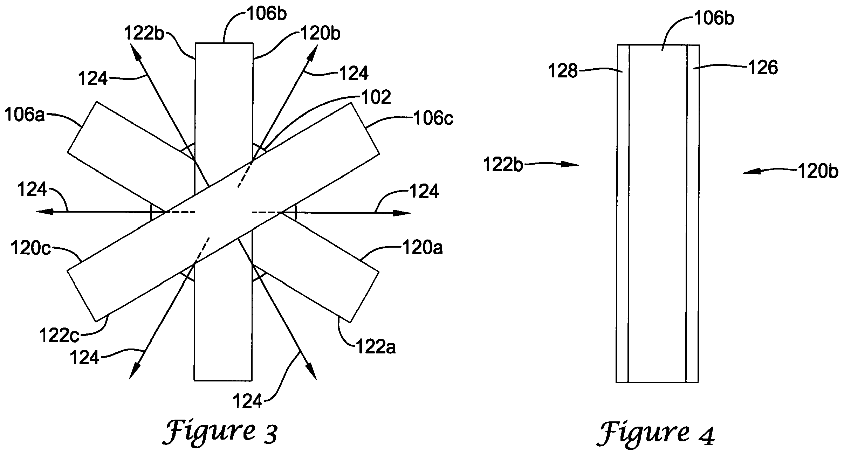

The system 100 further includes a first ultrasound transducer 106a, a second ultrasound transducer 106b, and a third ultrasound transducer 106c disposed adjacent to the distal end 104 of the elongate shaft 102. While the system 100 is described as having three ultrasound transducers 106a, 106b, 106c it is contemplated that the system 100 may include any plurality of transducers desired, such as, but not limited to: two, three, four, or more. The transducers 106a, 106b, 106c may be arranged in a longitudinal array. In some instances, the transducers 106a,106b,106c may extend parallel to the longitudinal axis of the elongate shaft 102. In other instances, the transducers 106a,106b,106c may be configured to curve or may be arranged in a curved formation to focus acoustic energy in a desired region. The first transducer 106a may have a proximal end 108 adjoining, or positioned adjacent to, the distal end 104 of the elongate shaft 102. The first transducer 106a may extend distally from a proximal end 108 thereof for a length L₁ and terminate at a distal end 110. The first transducer 106a may have a first side surface 120a defined by the length L₁ of the transducer 106a and a height H₁ of the transducer 106a. The transducer 106a also includes a second side surface 122a (see Figure 3) also defined by the height H₁ and length L₁ of the transducer 106a. The second side surface 122a is generally opposite and facing approximately 180° from the first side surface 120. The first and second side surfaces 120a, 122a are configured to radiate acoustic energy therefrom. The remaining surfaces (e.g., excluding surfaces 120a,122a) of the transducer 106a may form a perimeter of the transducer 106a.

The second transducer 106b may have a proximal end 112 adjoining, or positioned adjacent to, the distal end 110 of the first transducer 106a. The second transducer 106b may extend distally from a proximal end 112 thereof for a length L₂ and terminate at a distal end 114. The second transducer 106b has a first side surface 120b defined by the length L₂ of the transducer 106b and a height H₂ of the transducer 106b. The second transducer 106b also includes a second side surface 122b (see Figure 3) also defined by the height H₂ and length L₂ of the transducer 106b. The second side surface 122b is generally opposite and facing approximately 180° from the first side surface 120b. The first and second side surfaces 120b,122b are configured to radiate acoustic energy therefrom. The remaining surfaces (e.g., excluding surfaces 120b,122b) of the transducer 106b may form a perimeter of the transducer 106b.

The third transducer 106b may have a proximal end 116 adjoining, or positioned adjacent to, the distal end 114 of the second transducer 106b. The third transducer 106c may extend distally from a proximal end 116 thereof for a length L₃ and terminate at a distal end 118. The third transducer 106c has a first side surface 120c defined by the length L₃ of the transducer 106c and a height H₃ of the transducer 106c. The third transducer 106c also includes a second side surface 122c (see Figure 3) also defined by the height H₃ and length L₃ of the transducer 106c. The second side surface 122c is generally opposite and facing approximately 180° from the first side surface 120c. The first and second side surfaces 120c,122c are configured to radiate acoustic energy therefrom. The remaining surfaces (e.g., excluding surfaces 120c,122c) of the transducer 106c may form a perimeter of the transducer 106c.

In some cases, the length L₁,L₂,L₃, height H₁,H₂,H₃, and width W₁,W₂,W₃ of each of the transducers 106a,106b,106c may be identical such that the transducers 106a,106b,106c are of the same size. In other instances, the transducers 106a,106b,106c may be formed such that the length L₁,L₂,L₃, height H₁,H₂,H₃, and width W₁,W₂,W₃ may vary from one transducer to the next.

In some embodiments, the transducers 106a,106b,106c may be formed of a separate structure and attached to the elongate shaft 102. For example, the first transducer 106a may be bonded or otherwise attached to the elongate shaft 102. Likewise, the second transducer 106b may be bonded or otherwise attached to the first transducer 106a, and the third transducer 106c may be bonded or otherwise attached to the second transducer 106b. In some instances, the transducers 106a, 106b, 106c may include a ring or other retaining or holding mechanism (not explicitly shown) disposed around the perimeter of the transducers 106a,106b,106c. One or more of the transducers 106a, 106b, 106c may further include a post, or other like mechanism, affixed to the ring such that the post may be attached to the elongate shaft 102, an adjacent transducer, or other member. In some instances, the ring may be attached to the transducer(s) with a flexible adhesive, such as, but not limited to, silicone. However, it is contemplated that the ring may be attached to the transducer(s) in any manner desired.

In some instances, the first transducer 106a may be fixedly attached to the elongate shaft 102. Likewise, the second and third transducers 106b,c may be fixedly attached to the adjacent transducers. In such cases, when it is desirable to rotate the transducers 106a,106b,106c it may be necessary to rotate the entire elongate shaft 102. As will be discussed in more detail below, it may not be necessary to rotate the elongate shaft 14 360° as the transducers 106a,106b,106c can emit acoustic energy in multiple directions simultaneously. For example, the transducers 106a,106b,106c may ablate an entire perimeter of a vessel by only rotating the transducers 106a,106b,106c and/or elongate shaft 102 180° or oscillate through angles of +/- 90°.

In other instances, the first transducer 106a may be rotatably attached to the elongate shaft 102 such that the transducers (e.g., transducers 106a,106b,106c) can rotate independently of the elongate shaft 102. It is contemplated that in some instances, the second and third transducers 106a,b may be fixedly attached to the adjacent transducers 106a,106b,106c such that the transducers 106a,106b,106c move simultaneously with one another as the first transducer 106a is rotated. For example, the first transducer 106a may be coupled to a micromotor such that the first transducer 106a may be rotated and the second and third transducers 106a,b made to rotate with it. However, it is contemplated that each of the transducers 106a,106b,106c may be individually rotatable or any combination of fixed and rotatable desired.

The transducers 106a,106b,106c are formed from lead zirconate titanate (PZT). In some instances, the transducers 106a, 106b, 106c may include a layer of gold, or other conductive layer, disposed on the first surfaces 120, 120b,120c and second surfaces 122,122b, 122c over the PZT crystal for connecting electrical leads to the transducers 106a,106b,106c. In some instances, one or more tie layers may be used to bond the gold to the PZT. For example, a layer of chrome may be disposed between the PZT and the gold to improve adhesion. In other instances, the transducers 106a,106b,106c may include a layer of chrome over the PZT followed by a layer of nickel, and finally a layer of gold. It is contemplated that the layers may be deposited on the PZT using sputter coating, although other deposition techniques may be used as desired.

Figure 3 is a distal end view of the illustrative renal nerve modulation system of Figure 2. The transducers 106a,106b,106c may be positioned such that the transducers 106a,106b,106c direct ultrasound energy towards different radial positions around the target region. For example, the transducers 106a,106b,106c may be offset by 60° from one another such that ultrasound energy is directed in a relatively uniform pattern about the perimeter of the target region as indicated by arrows 124. However, it is contemplated that the transducers 106a,106b,106c may be offset by any angle desired (e.g., between about 0-180°). It is further contemplated that the angle of the offset may be determined by the number of transducers 106a,106b,106c present. For example, the angle of offset may be chosen such that ultrasound energy is relatively uniformly delivered about the perimeter of the vessel or target region, although this is not required. As will be discussed in more detail with respect to Figure 4, the transducers 106a,106b,106c are each be configured to direct ultrasound energy in two directions as illustrated by arrows 124. Thus, a system 100 including three ultrasound transducers 106a,106b,106c may ablate six different regions simultaneously. The may reduce the time required to perform a procedure as the system may not require repositioning or only require minimal repositioning (e.g., slight rotation to perform circumferential ablation). It is further contemplated that ablating multiple regions simultaneously may reduce cooling between ablation spots.

Figure 4 illustrates an illustrative cross-section of the second transducer 106b. While not explicitly shown, the first and third transducers 106a,106c are configured similarly to the second transducer 106b and include similar or the same structural features. The transducer 106b further includes a first matching layer 126 disposed on the first surface 120b and a second matching layer 128 disposed on the second surface 122b. In some instances, the matching layers 126,128 may provide acoustic impedance matching for efficient transmission. In some instances, the matching layer material may be selected such that acoustic impedance of matching layer 126,128 is equal to the geometric mean of the acoustic impedance of the transducer 106b (PZT) and adjacent media (e.g., blood). In some instances, the matching layers 126,128 may be a silver filled epoxy, although other materials may be used as desired. The matching layers 126,128 may each have a thickness approximately equal to one-fourth of the wavelength at the operating frequency, although other thicknesses may be used as desired.

It is contemplated that the first and second faces 120a,120b,120c/122a,122b,122c of the transducers 106a,106b,106c may take any shape desired, such as, but not limited to, square, rectangular, polygonal, circular, oblong. The acoustic energy radiated from the transducers 106a,106b,106c may take the shape of the transducers 106a,106b,106c (e.g., a rectangular transducer will generate a roughly rectangular beam of approximately equal size to the transducer). Thus, the shape of the transducers 106a,106b,106c may be selected based on the desired treatment and the shape best suited for that treatment. It is contemplated that the transducers 106a,106b,106c may also be sized according to the desired treatment region. For example, in renal applications, the transducers 106a,106b,106c may be sized to be compatible with a 6 French guide catheter, although this is not required. The length L of the transducers 106a,106b,106c may be sized to allow the transducers 106a,106b,106c to navigate the passageways to the desired treatment region. In some cases, the transducers 106a,106b,106c may be connected to the adjacent transducers 106a,106b,106c and/or elongate shaft 102 such that the distal end of the system 100 remains flexible to navigate tortuous passageways. For example, the transducers 106a,106b,106c may be connected in such a way that the connection points may bend and/or flex. In some instances, the transducers 106a,106b,106c may have a length L in the range of 0.5 to 10 millimeters (mm), 2 - 8 mm, or 3 - 6 mm. It is contemplated that, in certain applications, the transducers 106a,106b,106c may have a length less than 0.5 mm or greater than 10 mm. The height H of the transducers 106a,106b,106c may be dependent on the size of the guide catheter. For example, transducers 106a,106b,106c for use with a 6 French guide catheter may have a height H of 1.6 mm or less. In some instances, the transducers 106a,106b,106c may be used without a guide catheter. As such, the height H of the transducers 106a,106b,106c may be limited by the desired treatment region. The width W of the transducers 106a,106b,106c may be determined by the sum of the thicknesses of the PZT crystal, tie layer(s), conductive layer(s), and the matching layers. In some instances, the thickness of the PZT crystal may be approximately equal to one-half the wavelength at the operating frequency. In some cases, a transducer 106 including a PZT crystal and two matching layers 126,128 may have a thickness approximately equal one half the wavelength in the transducer 106 plus one half the wavelength in the impedance matching layer 126,128 at the operating frequency. However, the thickness of the transducer 106 may be less than or greater than this as desired.

While not explicitly shown, the transducers 106a,106b,106c may be connected to a control unit (such as control unit 18 in Figure 1) by electrical conductor(s). In some cases, the electrical conductor(s) may be disposed within a lumen of the elongate shaft 102. In other cases, the electrical conductor(s) may extend along an outside surface of the elongate shaft 102. The electrical conductor(s) may provide electricity to the transducers 106a,106b,106c which may then be converted into acoustic energy. The acoustic energy may be directed from the transducers 106a,106b,106c in a direction generally perpendicular to the first surfaces 120 and second surfaces 122 of the transducers 106a,106b,106c , as illustrated by arrows 124 in Figure 3. As discussed above, the acoustic energy radiated from the transducers 106a,106b,106c may take the shape of the transducers 106a,106b,106c (e.g., a rectangular transducer will generate a roughly rectangular beam having a size approximately equal to the size of the transducer). Thus, the acoustic energy may be radiated from the entire first and second surfaces 120,122 and not an isolated point.

In some instances, the transducers 106a,106b,106c may be connected to a control unit such that each of the transducers 106a,106b,106c is activated simultaneously. Simultaneous activation of the transducers 106a,106b,106c may provide more complete ablation as the tissue between two heated spots may heat more than when the two spots are heated in sequence or individually. For example, the transducers 106a, 106b, 106c may be configured as a geometric focusing array. In some instances, the transducers 106a, 106b, 106c be positioned having a curved geometry such that the ultrasound energy from the transducers 106a,106b,106c may overlap, at least in part. However, in some instances, the transducers 106a,106b,106c may be connected to a control unit such that each transducer 106a,106b,106c may be individually controlled. This may allow for any combination of the transducers 106a,106b,106c to be activated at any given time. For example, the transducers 106a,106b,106c may be configured as a phased array that may activate each of the transducers 106a,106b,106c such that the outside transducers (e.g., first and third transducers 106a,c in Figure 2) are activated first followed by the inside transducer(s) (e.g., second transducer 106b in Figure 2). This may allow the operator to focus ultrasound energy to a particular depth and location. However, these are merely exemplary of how the transducers 106 may be activated. The transducers 106a,106b,106c may be activated in any manner desirable for the particular treatment.

As discussed above with respect to the second transducer 106b, the transducers 106a,106b,106c are each formed with a matching layer 126,128 on two sides 120b,122b of the transducer 106b. Also as discussed above, the first and third transducers 106a,c include similar structures. In the absence of an air backing layer, acoustic energy may be directed from both the first side surface 120b and the second side surface 122b simultaneously. This may allow two sides of a vessel to be ablated simultaneously. As such, the transducers 106a,106b,106c may perform the desired ablation twice as fast as an ultrasound transducer which includes a backing layer. It is contemplated that the use of one-sided transducers may require twice as many transducers to perform a desired treatment compared to when double sided transducers (e.g., transducers 106a,106b,106c) are used.

In some instances, such as when circumferential ablation is desired, the transducers 106a,106b,106c and/or elongate shaft 102 may need to be rotated to complete the ablation. As two locations are being ablated simultaneously from each transducer 106a, 106b, 106c, (for a total of six locations), the transducers 106a,106b,106c may only need to be rotated 180° or oscillated +/- 90° to complete circumferential (360°) ablation adjacent each transducer 106a,106b,106c. If multiple radial ablation points are desired, the transducers 106a, 106b, 106c only need to be rotated half as many times as in single direction ablation. In some instances, the transducers 106a, 106b, 106c and/or elongate shaft 102 may be manually rotated (e.g., by a physician). Limiting the degree of rotation of the modulation system 100 may allow the transducers 106a,106b,106c to be fixedly secured to the elongate shaft 102 to further facilitate manual rotation. However, in other instances, the transducers 106a,106b,106c may be rotated continuously and/or automatically using a micromotor or other rotating mechanism. In some instances, when the transducers 106a,106b,106c are spun continuously, the speed of rotation may be reduced due to simultaneous ablation. However, as the adjacent transducers 106a,106b,106c may heat tissue adjacent to the region directly receiving the ultrasound energy, rotation may not be necessary to achieve ablation of the desired target region.

As the transducers 106a,106b,106c provide ultrasound energy along a length of the target region, it is contemplated that the ablation system 100 may not need to be longitudinally displaced to achieve the desired ablation. In some cases, such as when the desired target region is longer than the total length of the transducers 106a,106b,106c, the elongate shaft 102 may be longitudinally displaced to allow for ablation along a length of a vessel. For example, the modulation system 100 may be advanced within a vessel to a desired location and energy supplied to the transducers 106a,106b,106c. Once ablation at the location has been completed, the transducers 106a,106b,106c may be longitudinally displaced and energy again supplied to the transducers 106a,106b,106c. The transducers 106a,106b,106c may be longitudinally and/or radially displaced as many times as necessary to complete the desired treatment.

In some instances, it may be desirable to center the transducers 106a,106b,106c within the vessel being treated. Locating the transducers 106a,106b,106c in the center of the vessel may allow blood flow to pass by the first and second surfaces 120,122. This may provide passive cooling to the transducers 106a,106b,106c during operation. It is contemplated that a two-sided transducer may be cooled more efficiently than a one-sided transducer. The backing layer, which is absent in the present transducers 106a,106b,106c, may prevent the back side of the one-sided transducer from benefiting from the passive cooling supplied by the blood flow. Increased cooling (by allowing both surfaces to contact fluid flow) may increase the efficiency of the transducers 106a,106b,106c. As the power is relayed to the transducers 106a,106b,106c, the power that does not go into generating acoustic power generates heat. As the transducers 106a,106b,106c heat, they become less efficient, thus generating more heat. Passive cooling provided by the flow of blood may help improve the efficiency of the transducers 106a,106b,106c. As such, additional cooling mechanisms may not be necessary. However, in some instances, additional cooling may be provided by introducing a cooling fluid to the modulation system.

Blood flow, with or without additional cooling fluid, may be important for cooling the wall of the artery to prevent thermal damage to endothelium and smooth muscle layers. Damage to these layers can induce a physiologic reaction that may lead to scarring or stenosis or aneurism at the site of weakened smooth muscle. In the case of renal nerve modulation or ablation, the renal nerves lie adjacent to or behind the outside layer of the artery wall. This region may not benefit from blood cooling, and is heated by the beam of ultrasound energy. The heated zone penetrates to a depth that is determined by the acoustic power and tissue absorption of the ultrasound beam.

In order to keep the transducers 106a,106b,106c and distal catheter away from the artery wall to allow cooling of the artery wall and both sides 120,122 of the transducers 106a,106b,106c, a centering mechanism may be provided. In some instances, an inflatable balloon (not shown) may be provided. The inflatable balloon may be provided along the elongate shaft 102. When the desired treatment area is reached, the inflatable balloon may be expanded. It is contemplated that the inflatable balloon be sized and shaped to allow blood flow to continue to pass the transducers 106a,106b,106c. For example, the balloon may only partially occlude the vessel. Alternatively, in some cases, a spacing basket or struts may be used to center the system 100 within the vessel. It is further contemplated that in some instances two sided ultrasound ablation may utilize energy more efficiently than one-sided ablation. For example, allowing acoustic energy to radiate from two sides may reduce energy lost when the ultrasound waves are reflected off of a backing layer of a one-sided transducer. Increased cooling (by cooling at both sides) of the two-sided transducers 106a,106b,106c may also contribute to increased efficiency.

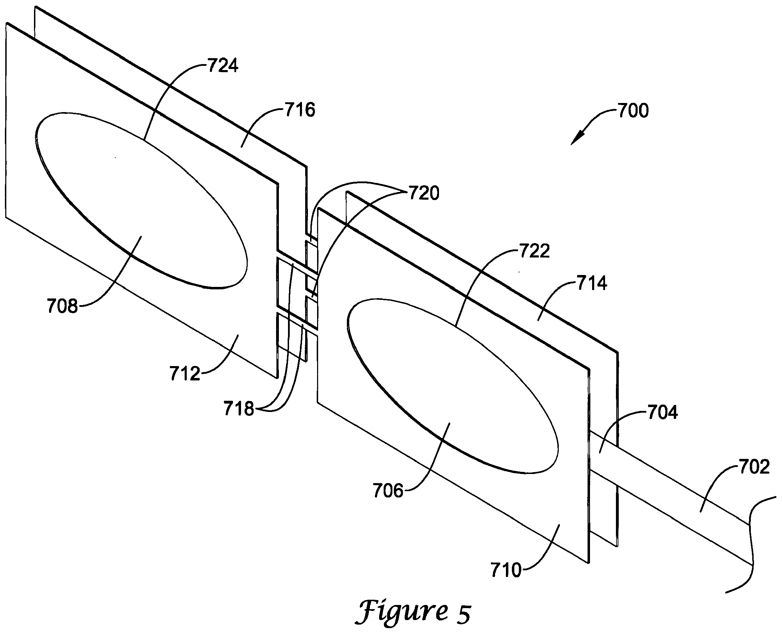

Figure 5 is a perspective view of a distal end of another illustrative renal nerve modulation system 700 that may be similar in form and function to other systems disclosed herein. The system 700 may include an elongate shaft 702 having a distal end 704. The elongate shaft 702 may extend proximally from the distal end 704 to a proximal end (not shown) configured to remain outside of a patient's body. The proximal end of the elongate shaft 702 may include a hub attached thereto for connecting other diagnostic and/or treatment devices or for providing a port for facilitating other interventions.

It is contemplated that the stiffness of the elongate shaft 702 may be modified to form modulation systems 700 for use in various vessel diameters. The elongate shaft 702 may further include one or more lumens extending therethrough. For example, the elongate shaft 702 may include a guidewire lumen and/or one or more auxiliary lumens. The lumens may be configured in any suitable way such as those ways commonly used for medical device. While not explicitly shown, the modulation system 700 may further include temperature sensors/wire, an infusion lumen, radiopaque marker bands, fixed guidewire tip, external sheath and/or other components may be incorporated to facilitate the use and advancement of the system 700 within the vasculature.

The system 700 may further include a first ultrasound transducer 706 and a second ultrasound transducer 708 disposed adjacent to the distal end 704 of the elongate shaft 702. While the system 700 is described as having two ultrasound transducers 706, 708 it is contemplated that the system 700 may include any plurality of transducers desired, such as, but not limited to: two, three, four, or more. The transducers 706, 708 may be arranged in a longitudinal array and may take any shape desired to accomplish the desired treatment. In some instances, the first transducer 706 may be mounted to the distal end 704 of the elongate shaft using a pair of metallic sheets 710,714. In some cases, the first metallic sheet 710 may define an opening 722. The first transducer 706 may be mounted to the first metal sheet 710 such that the face of the transducer 706 is aligned with the opening 722. While not explicitly shown, the second metallic sheet 714 may include a similar opening. Similarly, the second transducer 708 may be mounted between a pair of metallic sheets 712,716. In some cases, the first metallic sheet 712 may define an opening 724. The second transducer 708 may be mounted to the first metal sheet 712 such that the face of the transducer 708 is aligned with the opening 724. While not explicitly shown, the second metallic sheet 716 may include a similar opening. The metallic sheets 710,714/712,716 may be attached to the transducers 706,708 with a flexible adhesive, such as, but not limited to, silicone. However, it is contemplated that the metallic sheets 710,714/712,716 may be attached to the transducers 706,708 in any manner desired.

Each of the transducers 706,708 includes a first side surface configured to be aligned with the first openings 722,724 in the first metallic sheets 710,712. Each transducer 706,708 further includes a second side surface configured to be aligned with openings in the second metallic sheets 714, 716. The second side surfaces are positioned generally opposite and facing approximately 180° from the respective first side surfaces. The first and second side surfaces are configured to radiate acoustic energy therefrom. The acoustic energy can be directed from the transducers 706,708 in a direction generally perpendicular to the surfaces of the transducers 706,708. The transducers 706,708 include similar features and function in a similar manner to the transducers discussed with respect to Figures 2-4.

While the modulation system 700 is illustrated as having metallic sheets 710,714/712,716 positioned on either side of the transducers 706, 708, it is contemplated that only a single metallic sheet may be necessary to mount the transducers 706, 708 to the elongate shaft 702. In some instances, the metallic sheets 710,714/712,716 may be mounted to the transducers 706, 708 such that the metallic sheets 710,714/712,716 may be used for connecting electrical leads to the transducers 706, 708. It is contemplated that the metallic sheets 710,714/712,716 may be formed from or may include a layer of gold, or other conductive layer, disposed thereon. It is further contemplated that the first metallic sheets 710,712 may be separated from the second metallic sheets 714,716 by an insulator.

In some instances, the first metallic sheet 710 affixed to the first transducer 706 may be connected to the first metallic sheet 712 affixed to the second transducer 708 by one or more struts 718. While the first metallic sheets 710, 712 are illustrated as having two interconnecting struts 718, it is contemplated that there may be any number of struts desired, such as, but not limited to: one, two, three, four, or more. Similarly, the second metallic sheet 714 affixed to the first transducer 706 may be connected to the second metallic sheet 716 affixed to the second transducer 708 by one or more struts 720. In some instances, a first pair of metallic sheets 710, 712 and the interconnecting struts 718 and/or the second pair of metallic sheets 714, 716 and the interconnecting struts 720 may be stamped from a monolithic metallic sheet creating a unitary support structure. However, it is contemplated that the individual elements may be separately formed and affixed to one another using any method known in the art, such as, but not limited to: welded, brazed, soldered, glued.

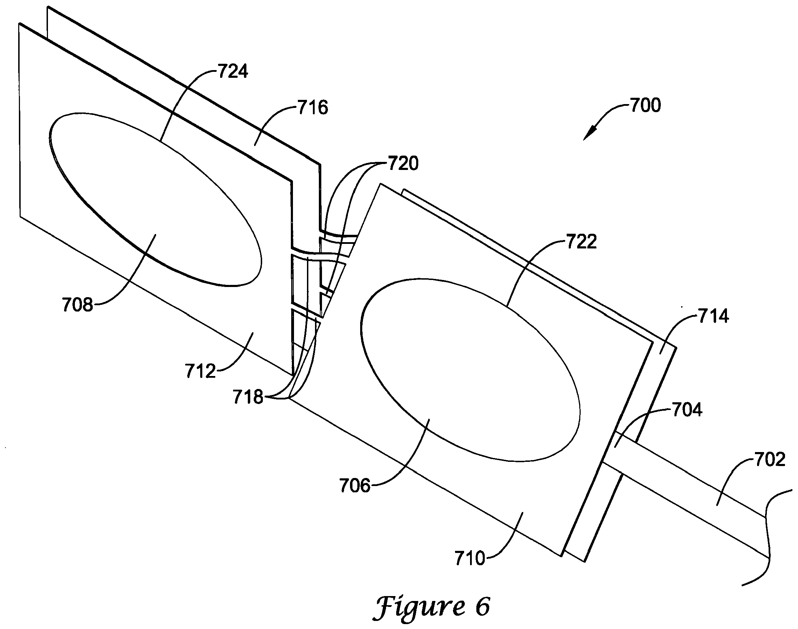

The interconnecting struts 718, 720 may flex or otherwise be configured to allow the transducers 706, 708 to be moved relative to one another. As can be seen in Figure 6, in some instances, torque or another external force may be applied to the elongate shaft 702 causing the struts 718,720 to flex or bend allowing the first transducer 706 to be positioned at an angle relative to the second transducer 708. This may allow for ultrasound energy to be directed at different radial positions about a vessel as desired by the user. The degree of offset may be determined by the amount of force exerted on the elongate shaft 702 and/or the flexibility of the interconnecting struts 718, 720. While not explicitly shown, the struts 718, 720 may be formed to provide additional flexibility between the transducers 706, 708. In some instances, the struts 718,720 may be formed with an undulating pattern to allow for additional flexibility and greater rotation. In other instances, the struts 718, 720 may be formed to provide enough rigidity to maintain the orientation of the transducers 706, 708 when they are in a flexed (e.g. as shown in Figure 6) orientation.

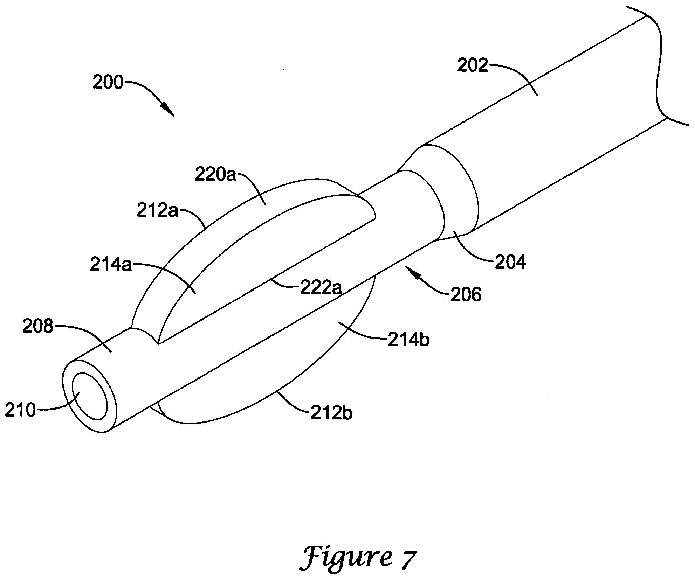

Figure 7 is a perspective view of a distal end of another illustrative renal nerve modulation system 200 that may be similar in form and function to other systems disclosed herein. The system 200 may include an elongate shaft 202 having a distal end region 204. The elongate shaft 202 may extend proximally from the distal end region 204 to a proximal end configured to remain outside of a patient's body. The proximal end of the elongate shaft 202 may include a hub attached thereto for connecting other diagnostic and/or treatment devices or for providing a port for facilitating other interventions.

It is contemplated that the stiffness of the elongate shaft 202 may be modified to form modulation systems 200 for use in various vessel diameters. The elongate shaft 202 may further include one or more lumens 210 extending therethrough. For example, the elongate shaft 202 may include a guidewire lumen 210 and/or one or more auxiliary lumens. The lumens may be configured in any suitable way such as those ways commonly used for medical device. While not explicitly shown, the modulation system 200 may further include temperature sensors/wire, an infusion lumen, radiopaque marker bands, a fixed guidewire tip, external sheath and/or other components may be incorporated to facilitate the use and advancement of the system 200 within the vasculature.

In some instances, an elongate tubular member 206 having an outer surface 208 and (e.g., which defines lumen 210) may extend distally from the distal end region 204 of the elongate shaft 202. The lumen 210 may be a guidewire lumen extending the entire length of the elongate shaft 202 such as in an over-the-wire catheter or a guidewire lumen extending only along a distal portion of the elongate shaft 202 such as in a single operator exchange (SOE) catheter.

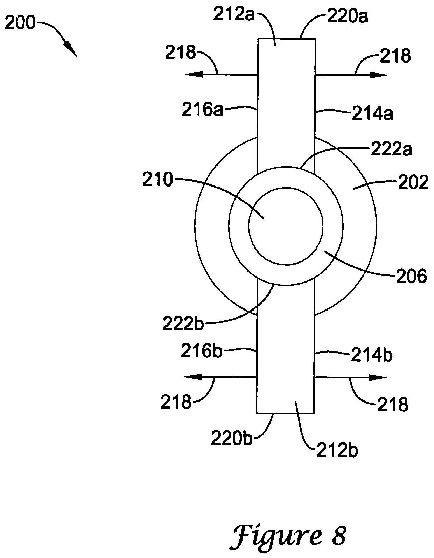

The system 200 may include one or more pairs of flat ultrasound ablation transducers 212a,212b arranged in a radial array and secured to the outer surface 208 of the elongate tubular member 206. It is contemplated, that in some embodiments, an odd number of transducers may be used. It is contemplated that the transducers 212a,212b may take any shape desired to accomplish the desired treatment. The transducers 212a,212b may be positioned parallel to a longitudinal axis of the elongate shaft 202. Referring now to Figure 8, which illustrates an end view of the illustrative modulation system 200 of Figure 7, the transducers 212a,212b may be secured to the elongate tubular member 206 along a first edge 222a,222b. In some embodiments, the transducers 212a,212b may be affixed directly to the elongate tubular member 206 by bonding or other suitable means. In some instances, the transducers 212a,212b may include ring or other retaining or holding mechanism (not explicitly shown) disposed about the first edge 222a and a second edge 222b. The ring may be attached to the transducer 212 with a flexible adhesive, such as, but not limited to, silicone. However, it is contemplated that the ring may be attached to the transducers 212a,212b in any manner desired. Affixing the transducers 212a,212b directly to the elongate tubular member 206 along the edge 222a,222b may allow a lumen (e.g., lumen 210) to extend to the distal end of the modulation system 200 without interfering with the ultrasound transducers 212a,212b. For example, if a tubular member is positioned along a side surface (e.g., a side surface 214a, 214b/216a,216b) of the transducers 212a,212b, the tubular member may absorb or otherwise interfere with the ultrasound energy emitted from the side surfaces 214a, 214b/216a,216b of the transducers 212a,212b.

As indicated above, each of the transducers 212a,212b has a first side surface 214a,214b and a second side surface 216a,216b (see Figure 8). The second side surfaces 216a,216b are positioned generally opposite and facing approximately 180° from the respective first side surfaces 214a,214b. The first and second side surfaces 214a,214b/216a,216b are configured to radiate acoustic energy therefrom. The acoustic energy may be directed from the transducers 212a,212b in a direction generally perpendicular to the surfaces 214a,214b/216a,216b of the transducers 212a,212b, as illustrated by arrows 218 in Figure 8. The transducers 212a,212b include similar features and function in a similar manner to the transducers discussed with respect to Figures 2-4.

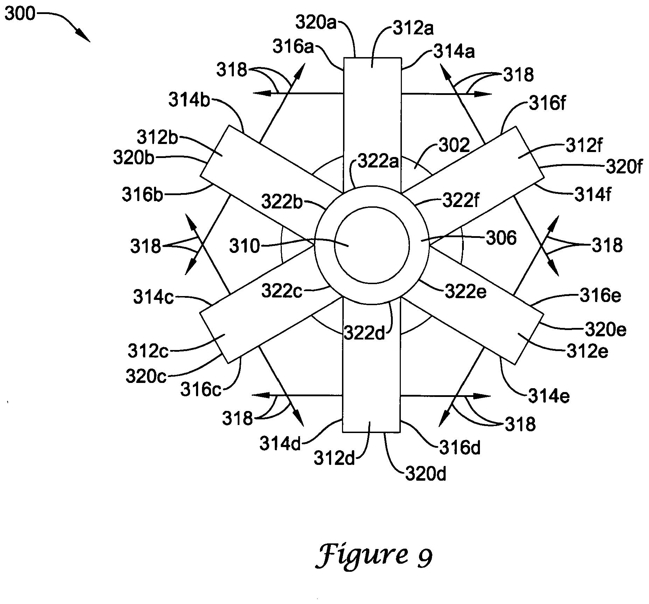

Figure 9 illustrates an end view of another illustrative modulation system 300. The modulation system 300 may include an elongate shaft 302 having an elongate tubular member 306 extending distally from a distal end region of the elongate shaft 302. The elongate shaft 302 and/or elongate tubular member 306 may include similar features to those discussed with respect to the system 200 illustrated in Figures 5 and 6. For example, the elongate tubular member 306 may define a lumen 310 for receiving a guidewire.

The modulation system 300 may include six transducers 312a-f radially spaced about the distal end of an elongate tubular member 306. In some instances, the transducers 312a-f may be evenly spaced about the elongate tubular member. However, the transducers 312a-f may be spaced as desired about the elongate tubular member 306. It is contemplated that the system 300 may include any plurality of transducers desired, such as, but not limited to two, three, four, five, or more. The transducers 312a-f may be secured to an outer surface of the elongate tubular member 306. It is contemplated that the transducers 312a-f may take any shape desired to accomplish the desired treatment. The transducers 312a-f may be positioned parallel to a longitudinal axis of the elongate shaft 302. The transducers 312a-f may be secured to the elongate tubular member 306 along a first edge 322a-f. In some embodiments, the transducers 312a-f may be affixed directly to the elongate tubular member 306 by bonding or other suitable means. In some instances, the transducers 312a-f may include ring or other retaining or holding mechanism (not explicitly shown) disposed about the first edge 322a-f and a second edge 320a-f.

Each of the transducers 312a-f has a first side surface 314a-f and a second side surface 316a-f. The second side surfaces 316a-f are positioned generally opposite and facing approximately 180° from the first side surfaces 314 a-f. The first and second side surfaces 314a-f,316a-f are configured to radiate acoustic energy therefrom. The acoustic energy may be directed from the transducers 312a-f in a direction generally perpendicular to the surfaces 314a-f,316a-f of the transducers 312a-f, as illustrated by arrows 318. The transducers 312a-f include similar features and function in a similar manner to the transducers discussed with respect to Figures 2-4.

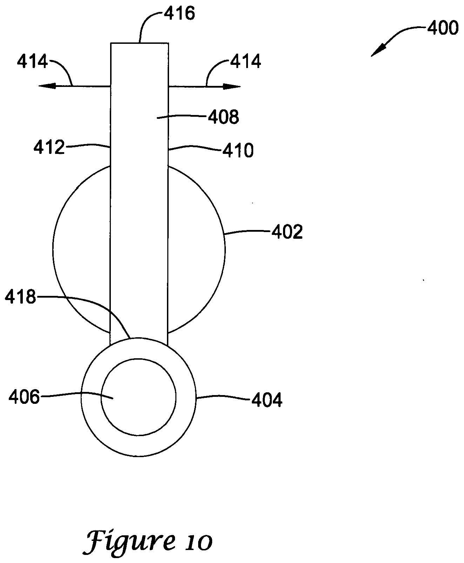

Figure 10 illustrates an end view of another illustrative modulation system 400. The modulation system 400 may include an elongate shaft 402 and an elongate tubular member 404 alongside an outer surface of the elongate shaft 402. The elongate tubular member 404 may extend distally beyond a distal end of the elongate shaft 402. In some cases, the elongate tubular member 404 may extend along the entire length of the elongate shaft 402. In other cases, the elongate tubular member 404 may only extend along a portion of the length of the elongate shaft 402. The elongate tubular member 404 may define a lumen 406 for receiving a guidewire. The elongate shaft 402 and/or elongate tubular member 404 include similar features to those discussed with respect to the system 100 illustrated in Figures 2-4.

The modulation system 400 may include a transducer 408 disposed adjacent a distal end of an elongate tubular member 404. It is contemplated that the system 400 may include any plurality of transducers 408 desired, such as, but not limited to two, three, four, five, or more. The transducer 408 may be secured to an outer surface of the elongate tubular member 404. It is contemplated that the transducer 408 may take any shape desired to accomplish the desired treatment. The transducer 408 may be positioned parallel to a longitudinal axis of the elongate shaft 402. The transducer 408 may be secured to the elongate tubular member 404 along a first edge 418. In some embodiments, the transducer 408 may be affixed directly to the elongate tubular member 404 by bonding or other suitable means. In some instances, the transducer 408 may include ring or other retaining or holding mechanism (not explicitly shown) disposed about the first edge 418 and a second edge 416.

The transducer 408 has a first side surface 410 and a second side surface 412. The second side surface 412 is positioned generally opposite and facing approximately 180° from the first side surface 410. The first and second side surfaces 410,412 are configured to radiate acoustic energy therefrom. The acoustic energy may be directed from the transducer 408 in a direction generally perpendicular to the surfaces 410,412 of the transducer 408, as illustrated by arrows 414. The transducer 408 includes similar features and functions in a similar manner to the transducers discussed with respect to Figures 2-4.

The present invention is defined by the appended claims.

## Claims

1. A system for nerve modulation, comprising:
an elongate shaft having a proximal end region and a distal end region; and
two or more ultrasound transducers disposed in an array adjacent to the distal end region of the elongate shaft, each of the two or more ultrasound transducers including a first side surface and a second side surface
wherein each of the two or more ultrasound transducers are configured to radiate acoustic energy from each of the first and second side surfaces simultaneously, **characterized in that** in each transducer, the second side faces a direction generally 180° opposite the first side surface and **in that**
each of the two or more ultrasound transducers comprises a lead zirconate titanate (PZT) crystal and a first matching layer on a first surface of the PZT crystal and a second matching layer on a second surface of the PZT crystal.

2. The system of claim 1, wherein the two or more ultrasound transducers are arranged in a longitudinal array.

3. The system of claim 2, wherein a proximal end of a first ultrasound transducer is secured to the elongate shaft and a proximal end of a second ultrasound transducer is secured to a distal end of the first ultrasound transducer.

4. The system of claim 3, further comprising a third ultrasound transducer secured to a distal end of the second ultrasound transducer.

5. The system of claim 4, wherein the first and second side surfaces of the first, second, and third ultrasound transducers are positioned at an angle to the first and second side surface of an adjacent transducer.

6. The system of claim 5, wherein the first and second side surfaces of the first, second, and third ultrasound transducers at an angle of 60° from the first and second side surface of an adjacent transducer.

7. The system of claim 1, wherein the two or more ultrasound transducers are arranged in a radial array.

8. The system of claim 7, further comprising an elongate tubular member extending distally from the distal end region of the elongate shaft.

9. The system of claim 8, wherein the two or more ultrasound transducers are affixed to an outer surface of the elongate tubular member.

10. The system of claim 9, wherein the two or more ultrasound transducers are evenly distributed about the outer surface of the elongate tubular member.

11. An intravascular nerve ablation system comprising: the system according to claim 1, wherein each of the plurality of ultrasound transducers comprises a gold coating on the first and second side surfaces.

12. The system of claim 11, wherein the transducers are configured to radiate acoustic energy from the first side surfaces and the second side surfaces simultaneously.

13. The system of any one of claims 11-12, wherein the plurality of transducers is arranged in a longitudinal array.

## Patentansprüche

1. System zur Nervenmodulation, mit:
einem länglichen Schaft, der einen proximalen Endbereich und einen distalen Endbereich aufweist; und
zwei oder mehreren Ultraschallwandlern, die in einer Anordnung benachbart zum distalen Endbereich des länglichen Schafts angeordnet sind, wobei jeder der beiden oder mehreren Ultraschallwandler eine erste Seitenfläche und eine zweite Seitenfläche aufweist, wobei jeder der beiden oder mehreren Ultraschallwandler konfiguriert ist, gleichzeitig Schallenergie von jeder der ersten und zweiten Seitenfläche abzustrahlen, **dadurch gekennzeichnet, dass** in jedem Wandler die zweite Seitenfläche in eine Richtung weist, die im Allgemeinen 180° zur ersten Seitenfläche entgegengesetzt ist und dass jeder der beiden oder mehreren Ultraschallwandler einen Blei-Zirkonat-Titanat- (PZT) Kristall und eine erste Anpassungsschicht auf einer ersten Oberfläche des PZT-Kristalls und eine zweite Anpassungsschicht auf einer zweiten Oberfläche des PZT-Kristalls aufweist.

2. System nach Anspruch 1, wobei die beiden oder mehreren Ultraschallwandler in einer longitudinalen Anordnung angeordnet sind.

3. System nach Anspruch 2, wobei ein proximales Ende eines ersten Ultraschallwandlers am länglichen Schaft befestigt ist und ein proximales Ende eines zweiten Ultraschallwandlers an einem distalen Ende des ersten Ultraschallwandlers befestigt ist.

4. System nach Anspruch 3, das ferner einen dritten Ultraschallwandler aufweist, der an einem distalen Ende des zweiten Ultraschallwandlers befestigt ist.

5. System nach Anspruch 4, wobei die erste und zweite Seitenfläche des ersten, zweiten und dritten Ultraschallwandlers unter einem Winkel zur ersten und zweiten Seitenfläche eines benachbarten Wandlers angeordnet sind.

6. System nach Anspruch 5, wobei die erste und zweite Seitenfläche des ersten, zweiten und dritten Ultraschallwandlers unter einem Winkel von 60° zur ersten und zweiten Seitenfläche eines benachbarten Wandlers angeordnet sind.

7. System nach Anspruch 1, wobei die beiden oder mehreren Ultraschallwandler in einer radialen Anordnung angeordnet sind.

8. System nach Anspruch 7, das ferner ein längliches Röhrenelement aufweist, das sich distal vom distalen Endbereich des länglichen Schafts erstreckt.

9. System nach Anspruch 8, wobei die beiden oder mehreren Ultraschallwandler an einer Außenfläche des länglichen Röhrenelements befestigt sind.

10. System nach Anspruch 9, wobei die beiden oder mehreren Ultraschallwandler gleichmäßig um die Außenfläche des länglichen Röhrenelements verteilt sind.

11. Intravaskuläres Nervenablationssystem, das aufweist:
das System nach Anspruch 1,
wobei jeder der mehreren Ultraschallwandler jeweils einen Goldüberzug auf der ersten und zweiten Seitenfläche aufweist.

12. System nach Anspruch 11, wobei die Wandler konfiguriert sind, gleichzeitig Schallenergie von der ersten und zweiten Seitenfläche abzustrahlen.

13. System nach einem der Ansprüche 11 bis 12, wobei die mehreren Wandler in einer longitudinalen Anordnung angeordnet sind.

## Revendications

1. Système pour une modulation nerveuse, comprenant :
une tige allongée ayant une zone d'extrémité proximale et une zone d'extrémité distale ;
et
au moins deux transducteurs ultrasonores disposés en un réseau adjacent à la zone d'extrémité distale de la tige allongée, chacun desdits au moins deux transducteurs ultrasonores comportant une première surface latérale et une deuxième surface latérale, chacun desdits au moins deux transducteurs ultrasonores étant prévus pour irradier simultanément une énergie acoustique depuis la première et depuis la deuxième surfaces latérales, **caractérisé en ce que**
dans chaque transducteur, la deuxième face est orientée dans une direction sensiblement opposée de 180° par rapport à la première surface latérale, et **en ce que** chacun desdits au moins deux transducteurs ultrasonores comprend un cristal titano-zirconate de plomb (PZT) et une première couche d'adaptation sur une première surface du cristal PZT et une deuxième couche d'adaptation sur deuxième surface du cristal PZT.

2. Système selon la revendication 1, où les au moins deux transducteurs ultrasonores sont disposés dans un réseau longitudinal.

3. Système selon la revendication 2, où une extrémité proximale d'un premier transducteur ultrasonore est fixée à la tige allongée, et une extrémité proximale d'un deuxième transducteur ultrasonore est fixée à une extrémité distale du premier transducteur ultrasonore.

4. Système selon la revendication 3, comprenant en outre un troisième transducteur ultrasonore fixé à une extrémité distale du deuxième transducteur ultrasonore.

5. Système selon la revendication 4, où la première et la deuxième surfaces latérales du premier, du deuxième et du troisième transducteurs ultrasonores sont positionnées suivant un angle par rapport à la première et à la deuxième surfaces latérales d'un transducteur adjacent.

6. Système selon la revendication 5, où la première et la deuxième surfaces latérales du premier, du deuxième et du troisième transducteurs ultrasonores sont positionnées suivant un angle de 60° par rapport à la première et à la deuxième surfaces latérales d'un transducteur adjacent.

7. Système selon la revendication 1, où lesdits au moins deux transducteurs ultrasonores sont disposés dans un réseau radial.

8. Système selon la revendication 7, comprenant en outre un élément tubulaire allongé s'étendant distalement depuis la zone d'extrémité distale de la tige allongée.

9. Système selon la revendication 8, où lesdits au moins deux transducteurs ultrasonores sont fixés sur une surface extérieure de l'élément tubulaire allongé.

10. Système selon la revendication 9, où lesdits au moins deux transducteurs ultrasonores sont répartis régulièrement autour de la surface extérieure de l'élément tubulaire allongé.

11. Système d'ablation nerveuse intravasculaire, comprenant :
le système selon la revendication 1,
chacun des transducteurs de la pluralité de transducteurs ultrasonores présentant un revêtement en or sur la première et la deuxième surfaces latérales.

12. Système selon la revendication 11, où les transducteurs sont prévus pour irradier simultanément une énergie acoustique depuis la première et depuis la deuxième surfaces latérales.

13. Système selon la revendication 11 ou la revendication 12, où la pluralité de transducteurs est disposée dans un réseau longitudinal.
